# EUROPEAN PATENT SPECIFICATION

(11) **EP 1 157 264 B1**
(45) Date of publication and mention of the grant of the patent: **21.04.2004**
(21) Application number: 00915860.1
(22) Date of filing: 25.02.2000
(51) Int. Cl.: G01N 1/34, B01L 3/00, C12Q 1/68

(54) **BIOCHEMICAL PURIFICATION DEVICES WITH IMMOBILIZED CAPTURE PROBES AND THEIR USES**
BIOCHEMISCHE REINIGUNGSGERÄTE MIT IMMOBILISIERTEN FANGSONDEN UND IHRE ANWENDUNGEN
DISPOSITIFS DE PURIFICATION BIOCHIMIQUE AVEC SONDES DE CAPTURE IMMOBILISEES ET LEURS UTILISATIONS

(30) Priority: 26.02.1999 US 121836 P
(43) Date of publication of application: 28.11.2001
(60) Divisional application: 03029149.6
(73) Proprietor: EXACT Sciences Corporation, Marlborough, Massachusetts 01752 (US)
(72) Inventor: ADAMS, Christopher, P., Somerville, MA 02144 (US); BOLES, T., Christian, Lexington, MA 02420 (US); WEIR, Lawrence, Hopkinton, MA 01748 (US); DHANDA, Rahul, K., Somerville, MA 02143 (US); KRON, Stephen, J., Oak Park, IL 60302 (US)
(74) Representative: Holdcroft, James Gerald, Dr.
(86) International application number: PCT/US2000/004771
(87) International publication number: WO 2000/050870

(56) References cited:
- EP-A- 0 776 700
- DE-A- 19 702 907
- DE-A- 19 751 968
- US-A- 5 453 382
- US-A- 5 632 957

## Description

### BACKGROUND OF THE INVENTION

The need to have highly purified DNA often arises in molecular biological research and applications, for example, in DNA sequencing. Current protocols for DNA sequencing require substantial purification of the DNA prior to automated analysis. Typically, the DNA is subjected to a replication protocol employing a replicating vector, for example, an M13 phage vector system, as well as DNA Polymerase, deoxynucleotides, primers, salts, and other replication constituents. The extension products that are formed during this replication step require further processing in order to obtain a relatively purified preparation containing only the extension products themselves. An often employed methodology for purifying the DNA extension products is ethanol precipitation. Ethanol precipitation is relatively effective in removing unincorporated nucleotides and salt from the preparation containing the extension products. It is also desirable to remove any template DNA and excess primers from the preparation prior to sequence analysis being performed on the extension products. However, precipitation is time consuming and requires great care in order to achieve consistent product yields.

A device is needed that would minimize the time factors involved, as well as provide a platform that would guarantee highly reproducible outcomes. Additionally, it would be advantageous to have a purification device that could sort the products of multiplexed preparative sequencing reactions. Additionally, it would be advantageous to have a purification device that could sort the products of multiplexed preparative sequencing reactions.

### SUMMARY OF THE INVENTION

The present invention pertains to devices comprising at least one purification unit according to claim 1 used to purify and/or concentrate a target molecule contained within a test sample and a method according to claim 8. Typically, the target molecule in a test sample will be a nucleic acid. These purified nucleic acids can be used in a variety of ways, including being subjected to nucleotide sequence analysis. Methods of using the device and kits containing the device are also provided.

The purification device of the present invention contains at least one purification unit. Alternatively, the device can contain a plurality of purification units, e.g., can be a multi-unit device. Each purification unit comprises a region that receives a test sample comprising a target molecule and a second region to purify and/or concentrate the target molecule, if present in the test sample. Typically, each purification unit includes three components which can be discrete or semi-discrete regions of the purification unit. The regions can also be referred to as chambers. Typically, the test sample moves from the first region to the second region and then to the third region. The first region receives a test sample that includes the target molecule along with non-target components such as contaminating proteins or buffer salts. The first region is referred to as the receptacle. The test sample is introduced into the receptacle. The second region is used to purify and/or concentrate the target molecule from other contaminating molecules contained in the test sample. Specifically, this separating region comprises an electrophoretic matrix, *e*.*g*., a polyacrylamide gel, that has one, or more, species or classes of immobilized capture probes within the electrophoretic medium. The immobilized capture probe specifically hybridizes with the target molecule. Thus, the target molecule is retained within the matrix in the separating region of the device while non-target, *e.g.*, contaminating components of the sample, pass through the separating region. For devices with purification units containing third regions, the electrophoretic medium separates the receptacle from the third region which is used to contain any or all molecules that pass through the second region of the device. This third region is called the collection chamber.

In an alternate embodiment, the purification device can include a plurality of purification units. In a preferred embodiment, the device is a microtiter plate and each purification unit is a microtiter well.

The purification device can also include a pre-purification unit which includes a receptacle, an electrophoretic medium, and a collection chamber. The electrophoretic medium separates the receptacle from the collection chamber.

In embodiments, the collection chamber includes an exit orifice. In a preferred embodiment, the exit orifice contains a semi-permeable membrane.

The device containing a single purification unit can contain a plurality of identical, or nearly identical, capture probes. The device containing a single purification unit can also contain a plurality of different capture probes. The device can also contain a plurality of capture probes, some portion of which are identical, or nearly identical, and some portion of which are different.

The device containing a plurality of purification units can contain a plurality of identical, or nearly identical, capture probes. The device containing a plurality of purification units can also contain a plurality of different capture probes. The device can also contain a plurality of capture probes, some portion of which are identical, or nearly identical, and some portion of which are different.

In another aspect, the invention pertains to a method of introducing a test sample containing the target nucleic acid molecule into the receptacle of a unit of a purification device including a receptacle and an electrophoretic medium including at least one immobilized capture probe selected to hybridize to the target nucleic acid molecule; subjecting the electrophoretic medium to an electric field resulting in the migration of the test sample through the medium, under conditions suitable for the target molecule in the test sample to hybridize to the capture probe, thereby forming a target molecule/capture probe complex, and for the remaining components of the test sample to migrate through and elute from the medium; and collecting the remainder of the test sample in the collection chamber.

In an embodiment, the method further includes the step of treating the electrophoretic medium to release the target molecule. In preferred embodiments, the target molecule can be released from the electrophoretic medium by raising the temperature of the electrophoretic medium to a temperature sufficient to denature the target molecule/capture probe complex, cleaving the chemical linkage which immobilizes the capture probes within the electrophoretic medium or increasing the electrophoretic field strength to a level sufficient to disrupt the target molecule/capture probe complex. In an embodiment, the target molecule is released into the receptacle.

The device used in the method further includes a collection chamber in which the collection chamber is separated from the receptacle by the electrophoretic medium.

In an additional embodiment, the method further includes a step of amplifying the target molecule.

In another emodiment, the method can result in a increase in the concentration of the target molecule.

In an alternate embodiment, the method can be used with a purification device which includes a plurality of purification units. In a preferred embodiment, a plurality of target molecules are purified simultaneously, *e*.*g*., the method is a multiplex method.

In a preferred embodiment, purification and concentration of a target nucleic acid molecule can occur in a single process step.

In another embodiment, the method can also include a pre-purification process step. In yet another embodiment, the test sample can be obtained from the collection chamber of a purification unit.

In another aspect, the invention pertains to methods for developing optimal conditions for methods of purifying target nucleic acids. In embodiments, the target nucleic acids are mutant nucleic acids known to be informative in disease detection. In embodiments, the conditions requiring optimization are those affecting capture of the target nucleic acids and elution of the target nucleic acids.

In another aspect, the invention pertains to a kit for preparing a target nucleic acid in a test sample for use in nucleic acid sequencing applications including a device for purifying a target nucleic acid from a test sample containing at least one purification unit, the purification unit including a receptacle and an electrophoretic medium containing at least one immobilized capture probe selected to hybridize to the target nucleic acid.

In an embodiment, the device of the kit further includes a purification unit including a collection chamber in which the collection chamber is separated from the receptacle by the electrophoretic medium.

In an alternate embodiment, the kit can include a device containing a plurality of purification units.

In an embodiment, the kit can further include a pre-purification unit including a receptacle, an electrophoretic medium, and a collection chamber, in which the electrophoretic medium separates the receptacle from the collection chamber.

In an embodiment, the target nucleic acid is a mutant nucleic acid useful in the detection of a human disease. In a preferred embodiment, the human disease is cancer.

The devices of the present invention permit quick, highly reproducible purification of nucleic acids from complex mixtures of molecules.

### BRIEF DESCRIPTION OF THE DRAWINGS

Figure 1 is a schematic representation of a method for purification of a target DNA for use in further analysis, e.g., DNA sequencing, using purification units of a device which are wells of microtiter plates.
Figure 2 is a schematic representation of a method for purification of a target DNA for use in further analysis, *e*.*g*., DNA sequencing, using a device with a purification unit that contains a collection chamber containing a semi-permeable membrane.

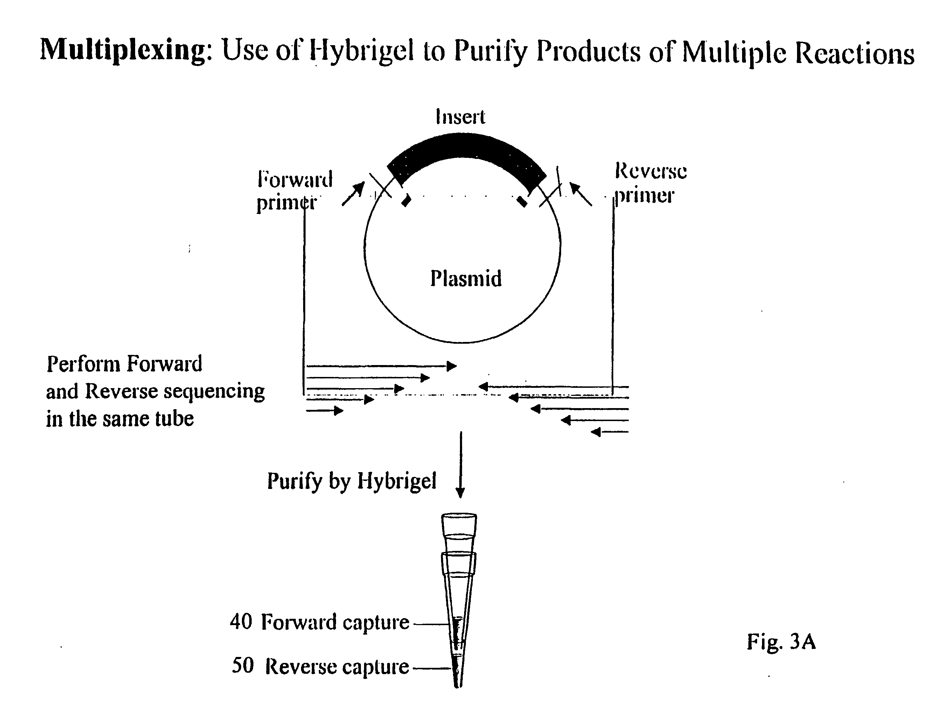

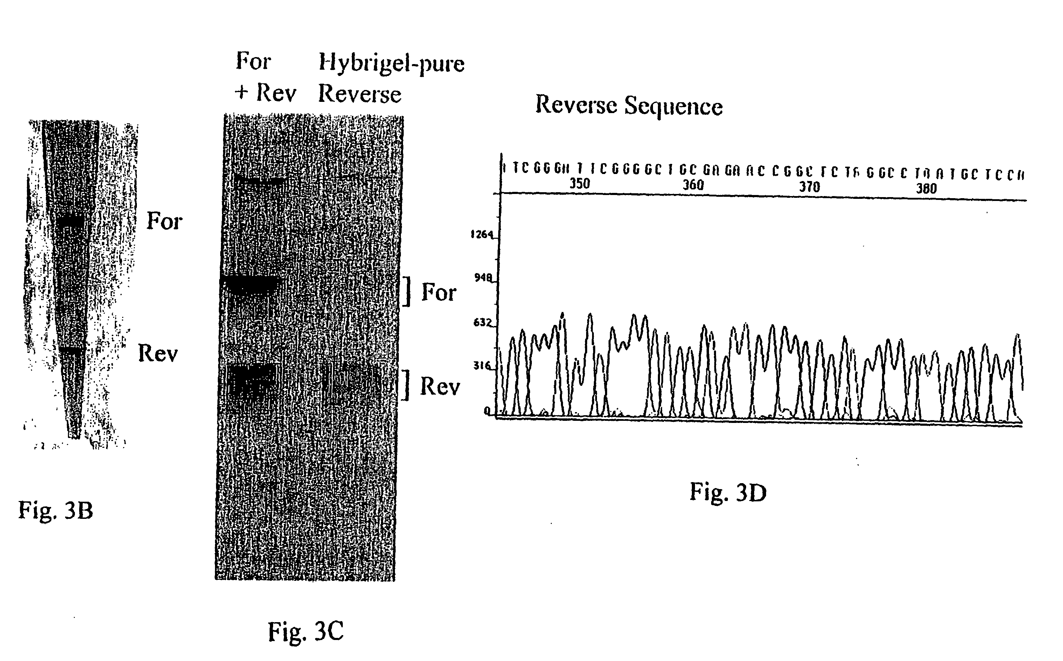

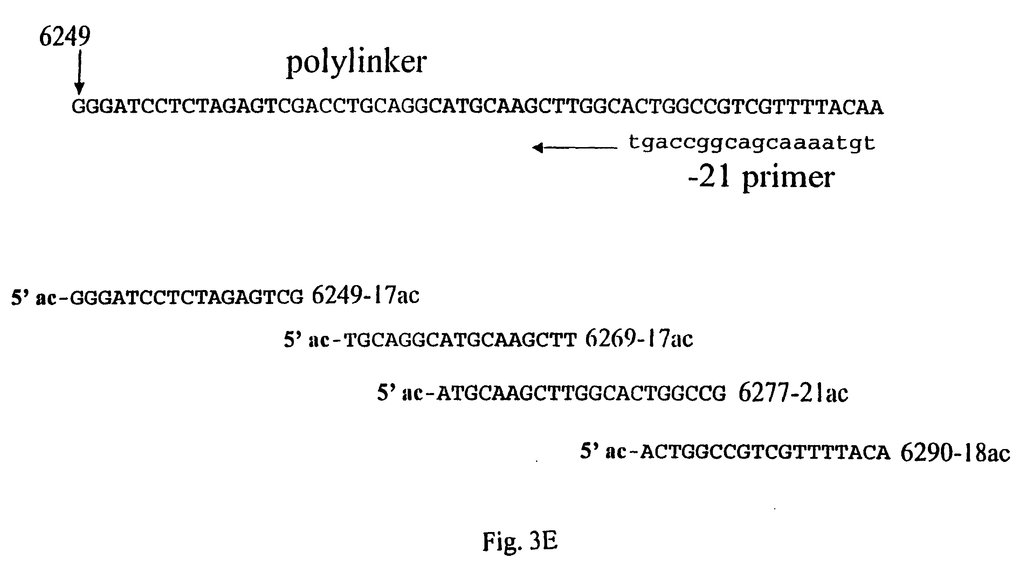

Figure 3A is a schematic representation of a method for purification of oligonucleotide products of multiple reactions performed in a purification unit in which a forward primer, a reverse primer, and a plasmid having an oligonucleotide insert to be sequenced are provided. The multiplex products of the amplification reaction are purified using stacked devices which contain an electrophoretic medium containing immobilized capture probes, *e*.*g*., probe-gel-tip devices, the upper device having a forward capture probe in the electrophoretic medium and the lower device having a reverse capture probe in the electrophoretic medium.
Figures 3B and 3C are photographs of the purification results achieved using the method depicted in Figure 3A. Forward replication products are separated from reverse replication products.
Figure 3D depicts the reverse sequence.
Figure 3E depicts the sequences used in the method depicted in Figure 3A.
Figure 4A is a schematic representation of a method using a device in which an electrophoretic medium which does not contain immobilized capture probes, *e*.*g*., a gel-loading-tip device, is used in tandem with a device containing an electrophoretic medium containing immobilized capture probes, *e.g.*, a probe-gel-tip device, to purify, and optionally concentrate, a DNA target molecule, *e.g.*, products of DNA sequencing reactions. A change in temperature is used to denature the hybridization complex and release the DNA target molecule.
Figure 4B is a photograph showing the distribution of the target molecule in the electrophoretic medium region after electrophoresis, and before elution.
Figure 5 is a photograph of an electrophoresis gel showing the distribution of components of samples taken 1) prior to purification in a device of the invention and 2) after hybridization and removal from the device.

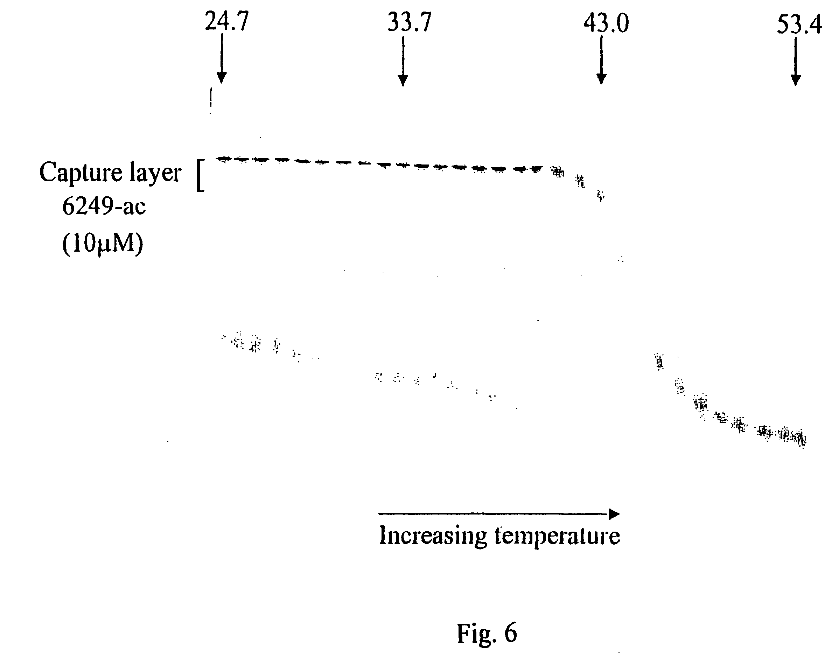

Figure 6 is a photograph of a vertical slab electrophoretic medium in which an electrophoretic medium containing immobilized capture probes, *e*.*g*., a probe-gel-layer, is sandwiched between an upper and a lower electrophoretic medium without immobilized capture probes, *e*.*g*., a gel-loading layer. The probe is uniformly distributed in the probe-gel-layer. A temperature gradient from 24.7° C to 53.4° C was provided from left to right during the electrophoresis of a target oligonucleotide having a dye tag.
Figure 7 is a photograph of a vertical slab electrophoretic medium with a plurality of lanes. Each lane is provided with a temperature gradient of from 23° C to 45° C from top to bottom. Each lane is provided with a capture probe of a different size. The complementary capture probe in Lane 1 is a 13 mer; Lane 2 a 15 mer; Lane 3 a 17 mer; Lane 4 a 19 mer; and Lane 5 a 21 mer. The same oligonucleotide with a dye tag is electrophoresed in each lane.

### DETAILED DESCRIPTION OF THE INVENTION

The present invention pertains to devices comprising at least one purification unit, used to purify and/or concentrate a target molecule contained within a test sample. The language "target molecule" and its plural "target molecules" is used essentially interchangeably throughout this application, and is intended to include any charged molecule which can be further purified or concentrated using the devices and methods of the invention.

Preferably, the target molecule will be a nucleic acid. The language "nucleic acid" is intended to include deoxyribonucleic acid, hereinafter "DNA", or ribonucleic acid, hereinafter "RNA". Such nucleic acids include, for example, DNA polymerase extension products generated by DNA sequencing procedures. These products can vary in length and often require further purification prior to DNA sequencing procedures. The purified target nucleic acids can be used in a variety of ways, including being subjected to further analysis, *e*.*g*., nucleotide sequence analysis.

The target nucleic acids can be contained in test samples containing a variety of components. Moreover, a test sample can originate from a variety of sources. For example, a test sample can originate from either a plant or an animal organ, tissue, or cell. A test sample can originate from a cell, tissue, or organ culture system. A test sample can originate from a cDNA or genomic library. DNA utilized in the test sample may be extracted from an organism found in a body sample, such as blood, urine, cerebrospinal fluid, tissue material and the like by a variety of techniques such as those described by Maniatis, *et al*., *Molecular Cloning: A* *Laboratory Manual,* Cold Spring Harbor, N.Y., pp. 280-281 (1982). A test sample can also be semi-purified, for example, a supernatant preparation from a tissue specimen can be used as a test sample.

The present invention provides purification devices useful to purify and concentrate a target molecule contained within a test sample. The devices of the invention include at least one purification unit containing discrete or semi-discrete regions. Typically, each purification unit includes at least three regions, also referred to herein as chambers or components.

The first region comprises a receptacle. The language "receptacle" is intended to include the region of a purification unit that can receive a sample.

The second region comprises an electrophoretic medium. The language "electrophoretic medium" or "electrophoresis medium" is a term of art and is intended to include all known media suitable for purifying and/or concentrating a target nucleic acid in a test sample. The electrophoretic medium of the device can be, for example, a polyacrylamide gel with oligonucleotide capture probes immobilized within the medium, e.g., the electrophoresis medium described in United States Patent No. 5,932,711 to Boles *et al*.. Capture probes can be designed to specifically interact with, and hybridize to, a target molecule contained within a test sample. The language "capture probe" and its plural "capture probes" is intended to include those probes that can hybridize to a target molecule, *e.g*, a target nucleic acid molecule.

The third region comprises a collecting chamber. The language "collecting chamber" is intended to include a region that contains the test sample after it has migrated through the electrophoresis medium. This chamber can be physically contiguous with the second region, or it can interface with the second region by being in close proximity with the second region. The third region can be detached from the second region, allowing for the emptying or filling of the collecting chamber and for the exchange of buffers.

The receptacle and the collecting chamber are physically separated from one another by the electrophoretic medium which forms a barrier between them.

The purification devices of the invention can be formed from many materials. Preferred materials are those most compatible with biological molecules, such as DNA and RNA. Materials like plastic, stainless steel, brass, ceramic, glass, silica, or various combinations of those materials can be used to form the purification devices. While the actual geometric configuration of the purification device is not critical to its operation, certain shapes can be beneficial for particular applications. For example, in some applications it is preferable for the circumference of the purification unit to differ in the regions containing the various components of the device.

Preferably, the purification device can be placed in contact with an electrical power source such that a voltage gradient can be established to facilitate migration of molecules through the electrophoretic medium contained within the second region. The electrical power source for purification can be detached and isolated from the actual purification device, or it can be integrated into the design of the purification device. An important feature of the device is to have the purification device capable of attaching, or being subject to, a voltage gradient produced by a power source. Electrodes from the power source, or receptacles for electrodes, can be an integrated feature of the purification device.

A test sample can be introduced into the purification device by placing it in the receptacle. An electric field, sufficient to allow the migration of the target molecule and other sample components through the separating electrophoretic medium, *e*.*g*., 0.1 to about 100-200 volts/cm, can be applied so that charged molecules in the receptacle can migrate through the electrophoresis medium toward the appropriate pole. Typically, the target nucleic acid molecules possess a negative charge and, therefore, migrate toward an anode. A target molecule can continue to migrate through the medium until it contacts an immobilized capture probe specific for that particular target molecule, *e.g*, a capture probe with which it will hybridize. A hybridization complex can form between the immobilized capture probe and the target molecule. Preferred capture probes are oligonucleotides modified with 5'-acrylamide groups that can co-polymerize within an electrophoresis gel, *e.g.*, a polyacrylamide gel. Target molecules can bind to complementary sequences contained within a capture probe, if there is substantial complementarity between the two molecules.

The language "substantial complementarity" is intended to include nucleic acid sequences of the capture probe that can hybridize to the corresponding target molecule. Such sequences are not required to reflect the exact nucleic acid sequence of the target molecule. Such sequences must only be sufficiently similar in identity of sequence to hybridize with the target molecule under specified conditions. For example, non-complementary bases, or additional nucleotides can be interspersed within sequences, provided that the sequences have sufficient complementary bases to hybridize therewith. Specific conditions of hybridization can be determined empirically by those of skill in the art.

For example, conditions of stringency should be chosen that significantly decrease non-specific hybridization reactions. Stringency conditions for nucleic acid hybridizations are explained in various textbook references, *e.g.*, Current Protocols in Molecular Biology, Ausubel, F.M., *et*. *al*., Vol. 1, Suppl. 26 (1991), the teachings of which are hereby incorporated by reference in their entirety. Factors such as probe length, base composition, percent mismatch between hybridizing sequences, temperature and ionic strength influence the stability of nucleic acid hybrids. Stringency conditions, *e.g.*, low, moderate, or high stringency, can be determined empirically, depending in part on the characteristics of the capture probe and the target molecule in the test sample.

The hybridization complex prevents further migration of the target molecule through the electrophoretic medium, but does not affect the continued migration of non-target molecules, thereby effectuating purification of the target molecule contained within the test sample. Non-target molecules can include, for example, proteins, peptides, sugars, salts, and nucleic acids.

The non-target molecules contained in the test sample can continue through the electrophoretic medium, and are effectively separated from the target molecule. Non-target molecules contained within the sample can pass through the gel and into a electrophoresis buffer contained in the collection chamber of the device. The buffer in the collection chamber can then be replaced with fresh electrophoresis buffer. The target molecule can be subsequently eluted from the capture probe and electrophoretic medium for subsequent analysis.

The language "elution" is intended to include melting the hybrid and moving the target in the direction of elution. Melting or disassociation of the target from the probe can be achieved by applying high temperature to the device, by altering the buffer conditions, *e.g.*, increasing pH or adding solvents such as formamide, or by applying high voltage.

For example, a sufficient voltage can be applied so that the hybridization complex formed between the target molecule and the capture probe is denatured, releasing the target molecule. The electric field can be applied using the same polarity as originally applied, thereby allowing for the continued migration of the released target molecule into the collecting chamber containing fresh electrophoresis buffer. Alternatively, the electric field can be reversed drawing the sample back into the sample well of the microtiter plate. The purified target molecule can now be accessed and subjected to further analysis, such as nucleotide sequence analysis. Alternatively, the target molecule can be introduced into an amplification reaction, for example, a polymerase chain reaction (PCR), a ligation chain reaction, a nucleic acid sequence based amplification or other equivalent procedures.

In one embodiment, the device contains a single purification unit, and is in the form of a microtube, or a disposable pipet tip, *e.g.*, Gilson or Eppendorf-type tips. In another embodiment, the device contains a plurality of purification units and is in the form of a microtiter plate. The microtiter plate can contain multiple wells, *e.g.*, ninety-six (96) wells, each of which forms a discrete purification unit. This embodiment facilitates the purification of multiple samples. If the device comprises a 96-well microtiter plate, then 96 discrete test samples can be introduced, *e.g.*, loaded onto the device. Microtiter plates containing other than 96 wells are also within the scope of this invention. The well of the microtiter plate can contain three regions. The first region of the well contains a receptacle for receiving a sample. The receptacle can preferably accommodate a volume of from about 5.0 µl to about 1000.0 µl, although larger samples can be used. The receptacle is located between the top surface of the well and the surface boundary formed by the electrophoretic medium. The electrophoretic medium comprises at least one species of capture probe, *e*.*g*., one oligonucleotide sequence, immobilized within the medium. Alternatively, multiple species of capture probes, *e*.*g*, multiple oligonucleotide sequences, can be immobilized within the medium (see, for example, USSN 08/971,845 entitled "Electrophoretic Analysis of Molecules Using Immobilized Probes" to Boles *et al*., filed August 8, 1997, the entire contents of which are hereby incorporated by reference.) In a preferred embodiment, the probe is covalently bound to the electrophoretic gel medium. The second region is located from the bottom surface of the receptacle to the top surface of the collection chamber, when it is contiguous with the first region.

In a further embodiment, a pre-purification unit can be utilized in conjunction with a purification unit of the invention. The electrophoretic medium of the pre-purification unit does not contain immobilized probes, rather, the unit is intended to provide an initial non-specific separation of the test sample which results in a partially purified sample. The test sample is placed in the receptacle of the pre-purification unit, preferably in electrophoresis buffer. In the presence of a voltage gradient, the sample is moved rapidly through the electrophoretic medium, *e.g.*, the gel-loading region. This separation region removes components based primarily upon size, in a sample where most of the molecules bear the same charge. Typically, the sample is drawn toward the anode, with the smaller molecules tending to move through the separation region most rapidly. Thus, the extension products pass through into a collection chamber more rapidly than the template that is retained in the electrophoretic medium.

The contents of the collection chamber from the pre-purification unit can then be further purified in a purification unit of a device of the invention. The partially purified sample is placed in contact with the top surface of the second region, usually by depositing the partially purified sample into the receptacle. The third region can be contiguous with the second region. The tip of the microtiter well can be removed by excision, or some other equally effective method. The orifice created by removing the basal tip of the well can be covered using a semi-permeable membrane. The semi-permeable membrane can have a molecular weight cutoff suitable for allowing contaminating sample components to pass through the membrane.

The microtiter plate can be associated with a power source such that an electric field can be applied to wells contained within the microtiter plate. The electric field can have any geometry with respect to the wells. Preferably, the electric field, when applied, will exit along the longitudinal axis of the wells such that charged molecules in the receptacle can migrate in a longitudinal direction through the electrophoresis medium contained within the second region. Once the sample is loaded, an electric field can be applied to the purification device such that charged molecules will migrate under the influence of the electric field.

Any electrophoresis medium, or matrix suitable for electrophoresis can be used in the devices of the present invention. Suitable matrices include acrylamide and agarose, both commonly used for nucleic acid electrophoresis. However, other materials may be used as well. Examples include chemically modified acrylamides, starch, dextrans and cellulose-based polymers. Additional examples include modified acrylamides and acrylate esters (see Polysciences, Inc., Polymer & Monomer Catalog, 1996-1997, Warrington, PA), starch (see Smithies, *Biochem*. *J*., 71:585 (1959), and product number S5651, Sigma Chemical Co., St. Louis, MO), dextrans (see Polysciences, Inc., Polymer & Monomer Catalog, 1996-1997, Warrington, PA), and cellulose-based polymers (see Quesada, *Current Opin*. *In Biotechnology*, 8:82-93 (1997)). Any of the polymers described can be chemically modified to allow specific attachment of capture probes to the electrophoretic medium for use in the present invention. A particularly preferred matrix is acrylamide.

A variety of capture probes can be used in the devices of the present invention. Typically, the capture probes of the present invention comprise a nucleic acid with a nucleotide sequence with substantial complementarity to a region of the nucleotide sequence contained within a target molecule, so that the target molecule can hybridize to the capture probe. The complementarity of the nucleic acid capture probes is only required to be sufficient to specifically bind the target molecule, and thus, to effectuate purification of the target molecule in a test sample. Probes suitable for use in the present invention include those formed from nucleic acids, *e.g.*, RNA, DNA, nucleic acid analogs, modified nucleic acids and chimeric probes of a mixed class comprising a nucleic acid with another organic component, *e.g.*, peptide nucleic acids. Capture probes can be single-stranded or double-stranded nucleic acids. Preferably, the length of the capture probe is at least 5 nucleotides, more preferably, the length is between 5 and 50 nucleotides, but the length can be up to several thousand nucleotides.

Nucleic acids useful for the probes of the invention include nucleic acids obtained by methods known in the art. These nucleic acids include substantially pure nucleic acids, nucleic acids produced by chemical synthesis and by combinations of biological and chemical methods, and recombinant nucleic acids. Both DNA and RNA can be used.

The language "nucleic acid analog" is intended to include nucleic acids containing modified sugar groups, phosphate groups or modified bases. Examples of nucleic acids having modified bases, include, for example, acetylated, carboxylated, or methylated bases, *e*.*g*., 4-acetylcytidine, 5-carboxymethylaminomethyluridine, 1-methylinosine, norvaline, or allo-isoleucine. Such nucleic acid analogs are known to those of skill in the art. One example of a useful nucleic acid analog is peptide nucleic acid (PNA), in which standard DNA bases are attached to a modified peptide backbone comprised of repeating N-(2-aminoethyl)glycine units (Nielsen, *et al*., *Science*, 254:1497-1500, (1991)). The peptide backbone is capable of holding the bases at the proper distance to base pair with standard DNA and RNA single strands. PNA-DNA hybrid duplexes are much stronger than equivalent DNA-DNA duplexes, probably due to the fact that there are no negatively charged phosphodiester linkages in the PNA strand. In addition, because of their unusual structure PNAs are very resistant to nuclease degradation. For these reasons, PNA nucleic acid analogs are useful for immobilized probe assays. It will be apparent to those skilled in the art that similar design strategies can be used to construct other nucleic acid analogs that will have useful properties for immobilized probe assays.

Probes containing modified oligonucleotides may also be useful. For instance, oligonucleotides containing deazaguanine and uracil bases can be used in place of guanine and thymine-containing oligonucleotides to decrease the thermal stability of hybridized probes. Similarly, 5-methylcytosine can be substituted for cytosine if hybrids of increased thermal stability are desired (Wetmur, *Critical Reviews in Biochemistry and Molecular Biology*, 26: 227-259(1991)). Modifications of the ribose sugar group, such as the addition of 2'-O-methyl groups can reduce the nuclease susceptibility of immobilized RNA probes (Wagner, *Nature*, 372:333-335 (1994)). Modifications that remove negative charge from the phosphodiester backbone can increase thermal stability of hybrids (Moody, *et*. *al*., *Nucleic Acids Res*., 17:4769-4782 (1989); Iyer, *et*. *al*., *J*. *Biol*. *Chem*., 270:14712-14717 (1995)).

Methods for attaching nucleic acids to form electrophoretic media containing immobilized probes are known to those of skill in the art and do not form part of the invention. Nucleic acids, modified nucleic acids and nucleic acid analogs can be coupled to agarose, dextrans, cellulose, and starch polymers using cyanogen bromide or cyanuric chloride activation. Polymers containing carboxyl groups can be coupled to synthetic capture probes having primary amine groups using carboiimide coupling. Polymers carrying primary amines can be coupled to amine-containing probes with glutaraldehyde or cyanuric chloride. Many polymers can be modified with thiol-reactive groups that can be coupled to thiol-containing synthetic probes. Many other suitable methods can be found in the literature (for a review see Wong, Chemistry of Protein Conjugation and Cross-linking, CRC Press, Boca Raton, FL (1993)).

Methods for covalently attaching the capture probes to polymerizable chemical groups have also been developed. When co-polymerized with suitable mixtures of polymerizable monomer compounds, matrices containing high concentrations of immobilized nucleic acids can be produced. Examples of preferred methods for covalently attaching nucleic acids to polymerizable chemical groups are found in Boles, *et*. *al*., United States Patent No 5,932,711, entitled "Nucleic Acid-Containing Polymerizable Complex," and in Rehman, *et*. *al*., *Nucleic* *Acid Res*., 27:649-655 (1999), the teachings of which are hereby incorporated by reference in their entireties.

For some embodiments of the device, composite matrices containing a mixture of two or more matrix forming materials, *e*.*g*., the composite acrylamide-agarose gel can be useful. These gels typically contain from about 2-5% acrylamide and 0.5-1% agarose. In these gels, the acrylamide provides the chief sieving function, but without the agarose, such low concentration acrylamide gels lack sufficient mechanical strength for convenient handling. The addition of agarose provides mechanical support without significantly altering the sieving properties of the acrylamide. In these embodiments, the nucleic acid can be attached to the component that confers the sieving function of the gel, since that component makes the most intimate contact with the solution phase nucleic acid target. Alternatively, a strengthening material or support onto which the gel matrix is bound may be provided (for example, see Jones, *et*.*al*., USSN 60/176,839; filed January 19,2000, entitled "Method of Detecting Nucleic Acid Using a Thin Gel Support and An Apparatus Therefor", the teachings of which are hereby incorporated by reference in their entirety).

Nucleic acids can be attached to particles which themselves can be incorporated into electrophoretic media. The particles can be macroscopic, microscopic, or colloidal in nature (see Polysciences, Inc., Polymer & Monomer Catalog, 1996-1997, Warrington, PA.*)*. Cantor, *et*.*al*., in United States Patent No. 5,482,863, describes a method for casting electrophoresis gels containing suspensions of particles. The particles are linked to the nucleic acids using methods similar to those described above, are mixed with gel forming compounds and are cast as a suspension into the desired matrix form.

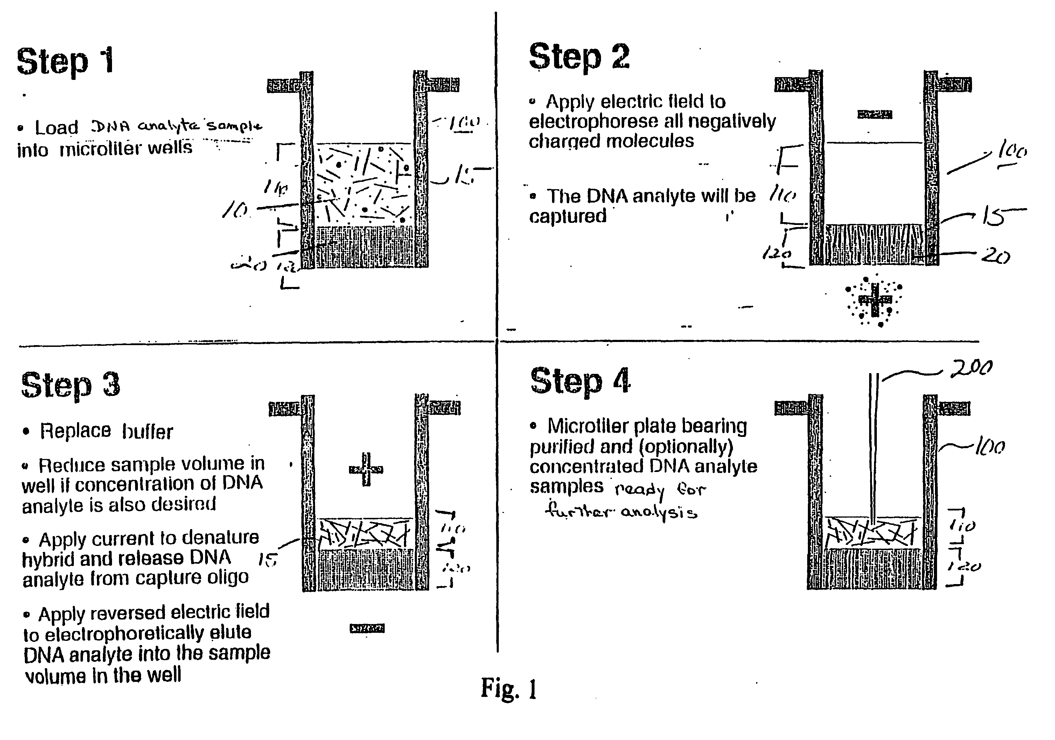

A review of the Figures will facilitate an understanding of the devices and methods of the present invention. Referring now to Figure 1, a method of using the inventive device is illustrated. In Step 1, a test sample 10 comprising DNA target molecules 15 along with DNA template, salts, monomers and buffer is placed into the first region 110 of a microtiter well 100. The test sample is in contact with the electrophoretic medium located in the second region 120 which comprises at least one capture probe 20. In Step 2, an electric field is applied to the contents of the microtiter well so negatively charged molecules can migrate through the electrophoresis matrix in the second region. The DNA target 15 is captured in the electrophoresis matrix 120 by the capture probe 20. In Step 3, the electrophoresis buffer is replaced with fresh buffer (preferably with a smaller volume than the volume of the DNA analyte sample) in the first chamber 110. Current is applied to denature the complex formed by the capture probe and the DNA target, thus releasing the DNA analyte target 15 from the capture probe. A reversed electric field is applied to electrophoretically elute the DNA analyte into the sample volume in the first region of the purification unit. In Step 4, removal of the DNA analyte from the first region of the purification unit is illustrated. The DNA analyte can be removed using a pipette 200 or other means, and the DNA analyte sample in buffer is then ready for further analysis such as, for example, DNA sequencing.

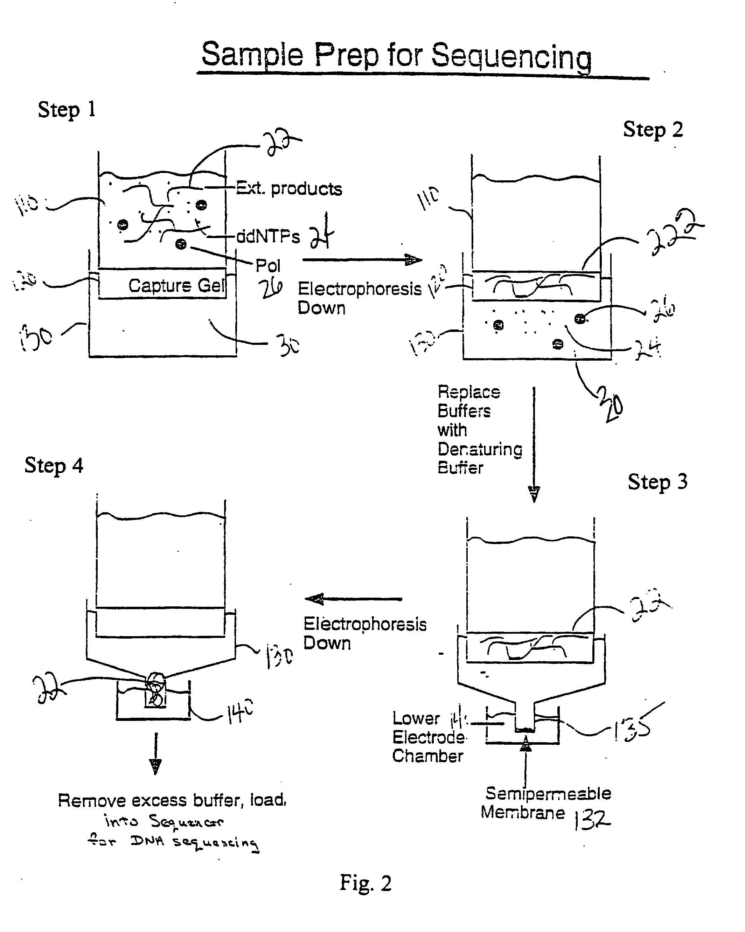

Referring now to Figure 2, a method for preparing a sample comprising primer extension products for sequencing is illustrated. In Step 1, a polymerase chain reaction is performed in buffer to provide extension products 22 in a sample solution. The sample solution, in addition to containing extension products, also contains ddNTPs 24, polymerase enzyme 26, salts and other components. In Step 2, the sample solution is placed in the receptacle 110 above the electrophoretic medium containing immobilized capture probes 120. The bottom of the electrophoretic medium is in contact with an electrophoresis buffer contained in the collection chamber 130. An electric field is established between the receptacle and the collection chamber 130. The ddNTPs 24, the polymerase enzyme 26 and the other non-target components of the test sample pass into the electrophoresis buffer 30, while the extensions products 22 hybridize with the capture probe forming a hybridization complex 222 captured within the gel matrix. The electrophoresis buffer is removed and denaturing buffer is placed both in the receptacle 110 and in the collection chamber 130. In Step 3, the collection chamber has a semi-permeable membrane 132 at the orifice at its base 135. The device is exposed to denaturing conditions to release the extension product 22 from the hybridization complex 222, allowing it to enter the collection chamber 130. The semi-permeable membrane prevents the extension products 22 from entering the lower electrode chamber 140 until excess buffer is removed. The extension products can then be loaded into an automatic sequencer for DNA sequencing.

In an alternate embodiment for providing a purified DNA sample for sequencing not depicted in the figures, the captured DNA sequence in the gel is introduced directly into a capillary on an automatic sequencer, thus eliminating the elution and recovery steps described above. For example, Acrydite beads can be made by preparing a Hybrigel solution (40% acrylamide (29:1 acrylamide monomer; bis-acrylamide) and 10 x TBE (90 mM Tris-Borate-EDTA buffer pH 8.3; reagents were purchased from Biorad Laboratories, Inc. Hercules, CA). A capture probe formed of an acrylamide phophoramidite derivative (Acrydite™ polymer available from Mosaic Technologies, Inc. Boston, MA) is then added. Polymerization is initiated by the addition of 10% ammonium persulfate and N, N, N', N'-tera-methyl-ethylenediamine (TEMED; BioRad, Hercules, CA). Droplets, from about 1.0 µl to about 10.0 µl, are pipetted into oil and polymerization is allowed to go to completion forming beads. A bead having the desired capture probe sequence can be placed in a well of a microtiter plate, in a tip, or in a similar non-interfering supporting device. A sample to be purified and concentrated is introduced onto the upper surface of the bead. Then, a volt gradient is established through the bead in the presence of electrophoresis buffer using platinum electrodes and a power source. The sample is allowed to electrophorese into the bead and capture of the sequence complementary to the capture probe sequence occurs. The bead in a small volume of buffer is then deposited on the electrode of the sequencer. The higher voltages used for sequencing are sufficient to denature the target/capture probe hybridization complex, and the DNA sequence moves out of the hybrigel bead through the liquid and into the capillary.

Referring now to Figure 3A, a method for purification of the products of a multiplexing reaction using an embodiment of the device is illustrated. In a multiplexing reaction, both strands of a double stranded oligonucleotide are replicated simultaneously in the same vessel using both forward and reverse primers. Thus, a plurality of oligonucleotide fragments are synthesized. A plasmid having a double stranded insert to be replicated is provided. The forward and the reverse primers each replicate their respective templates. Each generates a series of oligonucleotide fragments of varying length. Typically, overlapping sequences are produced as illustrated in Figure 3A. The oligonucleotide fragments generated by the forward primer are separated from the oligonucleotide fragments generated by the reverse primer using a purification unit of the device. A forward capture probe-gel-tip 40 is stacked above a reverse capture probe-gel-tip 50 and electrophoresis buffer is provided throughout. The test sample from the multiplex reaction is loaded into the receptacle of the forward capture probe-gel-tip 40 and is moved through both tips under the influence of the electrical field. Oligonucleotide fragments are captured by their respective capture probes as evidenced by the photographs of the gel shown in Figures 3B and 3C. Alternatively, prior to introducing the sample onto the probe-gel-tip stack, the sample may be electrophoresed *e*.*g*., pre-purified, through a gel-loading tip to remove unwanted components remaining from the multiplexing reaction.

The devices of the invention are particularly useful for selectively concentrating a mutant nucleic acid known to be informative in disease detection. In addition, purified target molecules find application in fingerprinting, tissue typing, forensic applications, maternity and paternity testing, fetal sex determination, as well as for quality control in agriculture, food, and pharmaceutical industries.

Comparing the DNA sequence found in an unknown sample to known DNA sequences, diagnostic procedures which rely on the identification of mutated sequences characteristic of a predisposition to cancer or of the potential for development of a genetic disease can be developed. For example, in a cancer detection assay, the mutant nucleic acid characterizing a cancerous cell can be found in the neoplastic tissue where the cancer originated, as well as in other biological samples depending upon the stage of the cancer, the type of cancer, and the tissue that is cancerous.

Specifically, certain patterns of deletions or point mutations, *e*.*g*., base changes, in gene sequences are characteristic of various stages of colorectal cancer. Stool samples have been used to detect colorectal cancer cells by distinguishing the mutant DNA sequences characteristic of the disease. For one example, see Vogelstein, *et*. *al*., United States Patent Nos. 5,380,645, 5,580,729 and 5,910,407. When a cancerous tissue metastasizes, mutant nucleic acid may be found in the blood and in the lymph nodes. However, compared to the quantity of the normal nucleic acid sequence, the mutant oligonucleotide sequence is present in very small quantities, typically in an amount less than 10% of the total present. The present invention provides a method for concentrating the mutant oligonucleotide to permit accurate sequence determination for disease detection.

A method for using the inventive device involves obtaining a blood sample, isolating nucleic acid, *e*.*g*, DNA, from the sample, and purifying and optionally concentrating the nucleic acid. This can be accomplished by placing the nucleic acid test sample obtained by extracting the blood into the receptacle of a device containing electrophoretic medium with a covalently bound capture probe sufficiently specific for the target nucleic acid sequence to allow hybridization with the capture probe. The target nucleic acid can be electrophoresed until it hybridizes to the capture probe. The nucleic acid can be removed from the probe, and optionally amplified to provide sufficient nucleic acid for sequencing. Alternatively, it can be directly sequenced. Concentration of the sample can be achieved by using a small amount of buffer when collecting the released target nucleic acid sequence from the hybridization complex.

Methods for developing optimal conditions for methods of purifying target nucleic acids are also included within the scope of the invention. Target nucleic acids, particularly target mutant nucleic acids known to be informative in disease detection can be purified most effectively when conditions affecting capture of the target nucleic acids and elution of the target nucleic acids are optimized.

Kits containing devices of the invention useful for practicing the described methods are also envisioned. A kit for accelerated preparation of DNA sequence products for capillary electrophoresis can include a container of gel-loading-tips and, a container of probe-gel-tips. It can also include electrodes sized to fit the tips, and a container of electrophoresis buffer and/or a power source to engage the electrodes.

A kit for accelerated multiplexing can include a container of probe 1-gel-tips (which may be a forward primer sequence complement) and a container of probe2-gel-tips (which may be a reverse primer sequence complement). Additionally, the kit can include a container of gel-loading tips, electrodes, a power source, and buffer or any combination of these components.

A kit for accelerating the detection of mutant DNA characteristic of the DNA sequence associated with cancerous tissue is also envisioned. Such a kit can comprise a container of probe-gel-tips, in which the probe sequence immobilized in the electrophoretic media complementary to a mutant DNA sequence known to be associated with a particular type of cancer.

Any of the kits can be configured to contain devices with a plurality of purification units.

### EXAMPLES

### EXAMPLE 1

### Purification of a Single DNA Product Complementary to M13mp18 Sequence

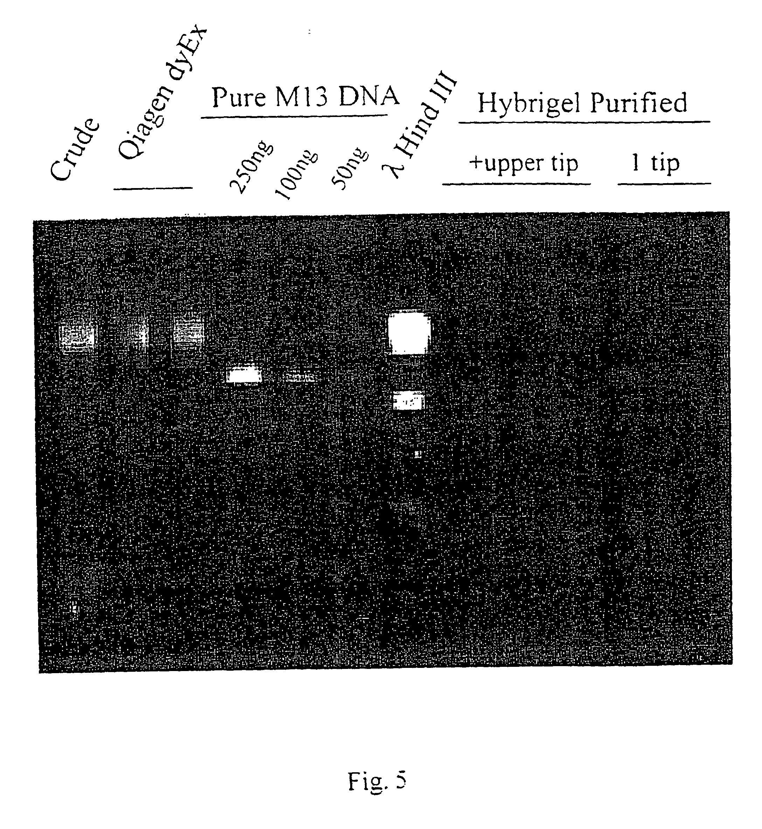

Figure 5 is a photograph of a gel showing the results of purification of a desired oligonucleotide sequence from a DNA sequencing reaction that included primers, salts, DNA template, unincorporated nucleotides, and dye terminators. First, the DNA for use in the sequencing reaction was purified by gel-loading tip to provide a crude sample, then the crude sample was purified by capture probe-gel-tip. Lane 1 shows the pattern provided prior to purification. Lane 2 provides the pattern seen after purification using a gel-loading tip. Removal of DNA template is demonstrated. Lane 3 shows the pattern seen after purification using a capture probe-gel-tip. Localization of the desired sequence in the absence of DNA template is demonstrated.

### A. Preparation of Separation Device With (Probe-Gel-Tip) and Without (Gel-Loading-Tip) Immobilized Capture Probe

The preparation of the probe-gel-tip, a purification device containing Acrydite™ oligonucleotide gel was performed as follows:

A Hybrigel solution was prepared from stock solutions of 40% acrylamide (29:1 acrylamide monomer:bis-acrylamide) and 10x TBE (90mM Tris-Borate-EDTA buffer pH 8.3; reagents were purchased from Biorad Laboratories, Inc.; Hercules, CA). The Acrydite™ oligonucleotide for the capture probe was synthesized using oligonucleotides (obtained from Operon Technologies, Inc.; Alameda, CA) and acrylamide phosphoramidite (Acrydite™ polymer available from Mosaic Technologies, Inc.; Waltham, MA) according to the methods disclosed in United States Patent No. 5,641,658 and the article of Kenney, Ray, and Boles, *BioTechniques* 25, 516-521 (1998). The Hybrigel contained 5% acrylamide (29:1), 1 x TBE and 10 uM Acrydite™ oligonucleotide capture probe having the following sequence:

5'-acrylamide GCT GAG ATC TCC TAG GG 3' (Probe 1) (SEQ ID NO: 1)

The selected capture probe for this example has a sequence that is complementary to a portion of the polylinker of vector M13mp18 which provides the target molecule, a product of a DNA sequencing reaction, but the capture probe does not include any of the primer sequence. When 10% ammonium persulfate and N, N, N', N'-Tera-methylethylenediamine (TEMED; BioRad, Hercules, CA) were added to the Hybrigel solution, polymerization was rapid (within 2 minutes).

To prepare the probe-gel-tip, 1.0 µl of 10% ammonium persulfate and 0.5 µl of TEMED were added to 200.0 µl of Hybrigel solution. 10.0 µl of the polymerizing Hybrigel oligonucleotide containing solution were quickly pipetted into a 200.0 µl tip (Fisher Scientific Co., Pittsburgh, PA) and allowed to polymerize. The probe-gel-tips were made 8 or 12 at a time using a multipipeting device (200 µl disposable pipette tips and multipipeting devices are available from Gilson, Middleton, WI). For storage, probe-gel-tips were ejected into microtube tips containing approximately 0.3 ml of 1x TBE. Care must be taken not to dislodge the gel from the tip. Then the probe-gel-tips were overlaid with 150.0 µl of 1x TBE.

Gel-loading tips were used to remove template DNA. Gel-loading tips were prepared containing acrylamide (29:1) only (*i*.*e*. without Acrydite™ capture probes). The gel-loading-tip was prepared as described above using a solution containing 5% acrylamide (29:1), 1 x TBE, made from stock solutions of 40% acrylamide (29:1 monomer: bis) and 10x TBE. 10% ammonium persulfate and TEMED were added to the solution and 200.0 µl of the final solution was pipetted into each tip. Gel-loading-tips may also be stored as described above.

### B. Preparation of a DNA Sequence Product and Capture Probes

PE-Applied Biosystems sequencing products were prepared following the protocol of PE Applied Biosystems BigDye Primer Cycle Sequencing Kit (available from PE-Applied BioSystems, Wellesley, MA) with the -21 M13 forward primer in a GeneAmp 2400 using the cycling conditions recommended by PE Applied Biosystems. Vector M13mp18 was used. A DNA segment having a known sequence was inserted after the primer site. Extension products were prepared. Capture probes were made to the region between the primer and the inserted DNA. The capture probes capable of hybridizing to extension products of the forward primer were selected. The capture probes comprise an oligonucleotide synthesized with Acrydite™ at the 5' end.

Alternatively, the DYEnamic ET Terminator Cycle Sequencing Kit from Amersham-Pharmacia may be used.

### C. Capture of DNA Sequence Extension Product

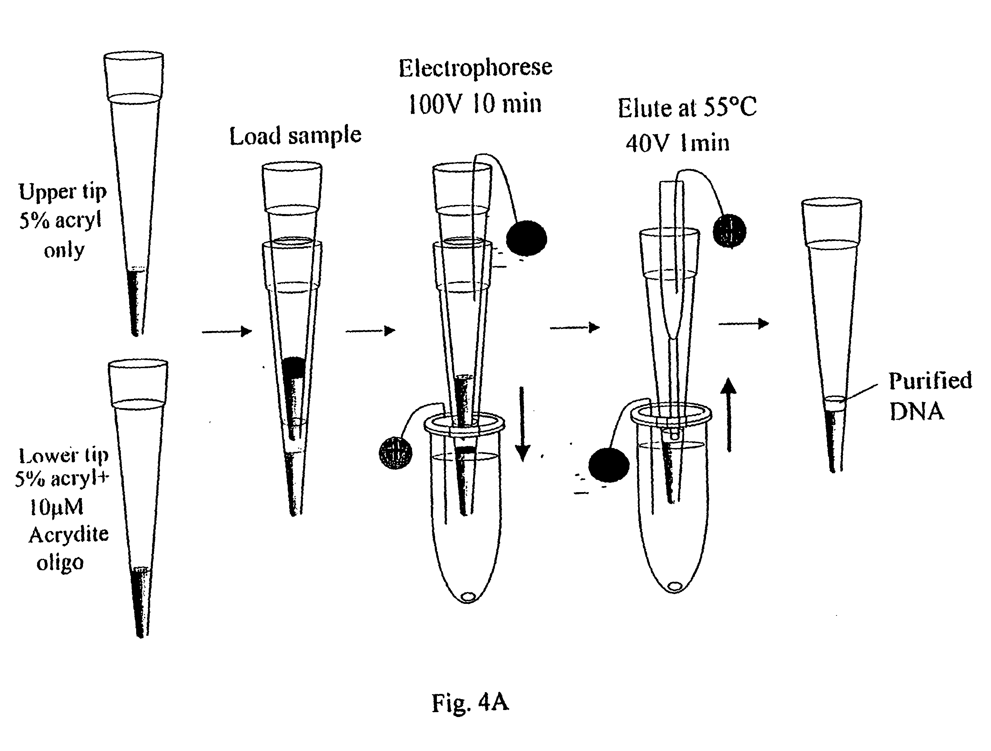

Electrophoretic capture and separation of a chosen extension product (target in test sample) were performed as follows: 10.0 µl of sequencing reaction solution (*i*.*e*. ½ of one reaction) that contained primers, salt, unincorporated nucleotides, dye terminators, template DNA, and the target DNA extension products synthesized from the template DNA were added to 1.0 µl of 10x ficoll loading buffer (35% ficoll 400, 0.1% Bromophenol blue, 0.1% xylene cyanol, 100mM EDTA) to provide a sample solution. A volume, approximately 200 µl, of electrophoresis buffer was layered onto the surface of the gel in the gel-loading-tip, while a smaller volume, sufficient to keep the surface wet and provide electrical contact) was layered onto the surface of the probe-gel-tip. The sample solution was layered onto the surface of the gel which is in a gel-loading-tip, (*i*.*e*. without Acrydite™ capture probe), to remove template DNA. This tip was stacked above a probe-gel-tip, a Hybrigel containing tip, as shown in Figure 4A. A small volume of electrophoresis buffer was layered onto the surface of the gel in the probe-gel-tip and the gel-loading-tip was placed in contact with the electrophoresis buffer. Thus, a liquid connection was formed between the two gels allowing for an electrical connection when the electrodes and power source were in place.

After the sample solution was loaded onto the surface of the gel of the gel-loading-tip, both tips were placed into microtubes (1.5 ml) containing 1x TBE, electrophoresis buffer. Platinum electrodes were placed in the electrophoresis buffer above and below the stacked gel tips and a voltage of 100V was applied for 10 minutes. The upper electrode was connected to the negative lead of the power supply, while the lower electrode was attached to the power supply's positive electrode. The gel-loading tip thus provided a partially purified sample solution for introduction into the probe-gel-tip. (At this point, the gel-loading tip can be discarded.) The smaller DNA fragments pass into the buffer and then into the probe-gel-tip more rapidly than the DNA template and dye which are retained in the gel-loading-tip.

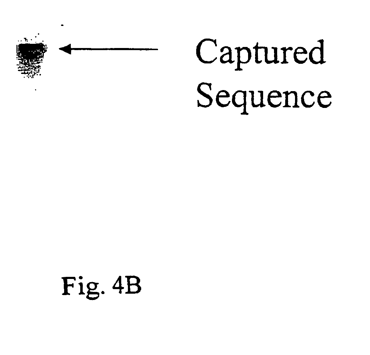

The electrophoresis buffer remaining above the probe-gel-tip gel surface was removed. The electrophoresis buffer was replaced with 4 µl of formamide loading dye (5:1 deionized formamide, 25mg/ml blue dextran, 25mM EDTA). The temperature in the gel in the probe-gel-tip was raised to 55 ° C to facilitate detachment of the target oligonucleotide sequence from the capture probe by placing the microtube tips containing the lower buffer reservoir into a drybath. (VWR). A clean second get-loading tip with a 30% acrylamide gel containing 5% acrylic acid was lowered into the formamide loading dye. A platinum electrode was placed in the top gel-loading tip. The direction of the current was reversed to drive the oligonucleotide released from the hybridization complex upwards. One (1) minute at 40V was sufficient to drive the oligonucleotide out of the probe-gel-tip and into the formamide loading dye. A 4.0 µl sample was removed by pipet and retained for sequence analysis. After electrophoresis, the gel in the probe-gel-tip was visualized using a Molecular Dynamics Fluorimager 595. The results shown in Figure 4B demonstrate that capture occurs at the upper surface of the gel in the probe-gel-tip.

### D. Analysis of Sequence Purification by Hybrigel Assay

Glass plates for a vertical polyacrylamide minigel (10 x 10 cm, 0.75 mm spacers) were assembled and the sandwich was filled approximately half way with 20% acrylamide (29:1; Bio-Rad), 1x TBE (90 mM Tris-borate buffer, pH 8.3, 2 mM EDTA). Polymerization was initiated by inclusion of 10% aqueous ammonium persulfate (APS) and TEMED at 1/100th and 1/1000th gel vol, respectively. For gels containing one capture layer, 600 µl of gel solution (20% polyacrylamide, 1x TBE, 4 µl 10% APS and 4 µl 10% TEMED) containing Acrydite™-labeled oligonucleotide at a final concentration of 10 µM were polymerized. After polymerization of the capture layer, the remaining space in the plate sandwich was filled with a 5% gel. This composite gel was then assembled in a minigel apparatus containing 1x TBE and subjected to electrophoresis at 100-150 V for ∼45 min. After electrophoresis, the gel was visualized using a Molecular Dynamics Fluorimager 595. The results confirm that the sequence captured by the Hybrigel probe is the complement of the template DNA.

### E. Automated Sequencing of the Captured Oligonucleotide

Following the procedures described above and analyzing the oligonucleotide sequence purified by the inventive device with an automated sequencer, repeated experiments with standard vectors have demonstrated that the accuracy of sequencing of the first 500 nucleotides approaches 100%. The readable sequence extends to at least 750 nucleotides.

### EXAMPLE 2

### Simultaneous Separation of Multiple DNA Sequence Products

This example demonstrates that the inventive device and method is useful for sequencing an oligonucleotide insert replicated in a plasmid. Both forward and reverse primers are used as illustrated in Figure 3A. A plasmid with a large insert was sequenced simultaneously using two primers in one reaction vessel. Two probe-gel-tips each containing a different capture probe were arranged in tandem. The probe sequences were designed based upon the forward and reverse primers used. From the slab gel illustrated in Figure 5, purification of the two oligonucleotide targets in the test sample as compared to the crude product is demonstrated. Note that the sequence of the reverse product (right) shows no contamination with the forward sequence. Note also that DNA template contamination is removed.

### A. Preparation of Separation Device With and Without Immobilized Capture Probe

### Gel-loading tips were prepared as described in Example 1.

Probe-gel-tips were prepared as described in Example 1, except that one was provided with a forward primer probe (Probe 2) and the other was provided with a reverse primer probe (Probe 3) as shown in Figure 3A. Thus, two separate Hybrigel probe-gel-tips were made, one with acrydite oligonucleotide 6269-17ac (SEQ ID NO: 2) and the other with acrydite olgonucleotide 6231-19ac (SEQ ID NO: 3). Plasmid p698 which is plasmid vector pGEM3Zf(-) (Promega Biotech, WI) with a 3.8 kb insert in the Xba I site of the polylinker was used. The two probes derive from sequence on either side of the Xba I site within the polylinker. Probe 6269-17ac is complementary to, and therefore capable of, capturing sequence products made using the reverse primer. Similarly, probe 6231-19ac can capture sequence from the forward primer.

6269-17ac 5'acrylamide –TGCAGGCATGCAAGCTT (SEQ ID NO: 2)

6231-19ac 5'acrylamide –GGGTACCGAGCTCGAATTC (SEQ ID NO: 3)

Thus, each oligonucleotide primer probe is synthesized with Acrydite at the 5' end and is capable of hybridizing to an extension product. Each capture probe sequence is both complementary to an extension product sequence and to the primer. Each capture probe sequence is specific for a particular vector derived from the region between the primer site and the insert DNA.

### B. Purification of Products of Multiple Reactions

Probe2-gel-tip was placed in tandem (stacked) with a probe3-gel-tip as is illustrated in Figure 3A. Running electrophoresis buffer was placed on the upper surface of probe3-gel-tip to provide electrical contact and to prevent drying. The test sample from a multiplex sequencing reaction was electrophoresed through both probe2-gel-tip and probe3-gel-tip. Figure 3B illustrates capture in the two separate tips by the two distinct probes.

### EXAMPLE 3

### Elution of Target from Capture Probe using a Temperature Gradient

The following method can be used to determine optimal temperature for capture and elution.

The stability of the hybridization complex is dependent on temperature. A vertical slab gel containing a layer of Acrydite™ capture probe sandwiched between layers of gel without capture probe was made. The gel for the upper and lower layers was that used for the gel-loading-tip. The Acrydite probe layer was made as described in Example 1 for the probe-gel-tip using the capture probe sequence 6249-17ac (SEQ ID NO: 4). The sample in this case was a fluorescent oligonucleotide with a complementary sequence to 6249-17ac. The same sample was loaded in each well. The whole gel was subjected to a temperature gradient using an aluminum backplate and two water baths. The gel temperature on the left is 23 ° C. The temperature increased across the gel up to 53° C on the right. At low temperature the target was efficiently captured at the top of the capture layer. As the temperature was increased, target capture was inhibited until the sample runs right through the layer. The transition temperature, *i*.*e*., the temperature at which the target stops ceases to be captured is related to the T_{m.} Temperatures below this transition temperature are useful for capture, while those above this transition temperature are useful for elution.

### EXAMPLE 4

### Temperature and Capture Probe Size Dependence of Sequence Elution

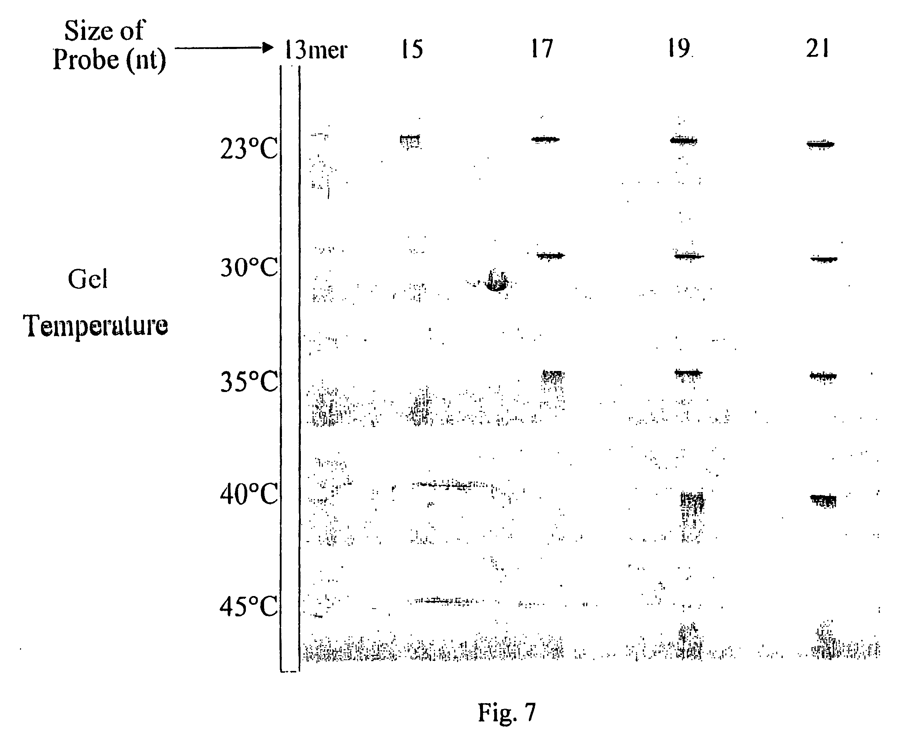

To define temperature conditions for capture and elution of sequencing products the experiment shown in Figure 7 was performed. This experiment demonstrates that the temperature of elution is affected by the size of the capture probe. The temperature of elution was shown to be affected by the size of the capture probe. The five panels shown in Figure 7 show the same vertical slab gel run at five different temperatures starting with 23° C, wherein five different capture probes were used. The number of bases in each probe sequence was 13, 15, 17, 19, and 21 nucleotides as indicated in Figure 7 (from left to right). An M13 sequencing reaction (Dynamic™ ET) was loaded in each lane. The 13 mer did not capture well at 23° C, while all the others did. At 30° C 15 mer released the sequence, while the 17mer started to release at 35° C, and so on until at 50° all target sequences came off. These results illustrate temperature conditions for capture and elution of sequencing products.

The 17 mer Acrydite™ probe was chosen as the capture probe in the standard procedure with elution occurring at 45° C.

While this invention has been particularly shown and described with references to preferred embodiments thereof, it will be understood by those skilled in the art that various changes in form and details may be made therein without departing from the scope of the invention encompassed by the appended claims.

## Claims

1. A device for purifying a target nucleic acid molecule from a test sample comprising at least one purification unit, the purification unit including a conduit comprising:
(a) a first region including a receptacle for receiving a sample;
(b) a second region including an electrophoretic medium with at least one immobilized capture probe selected to hybridize to the target nucleic acid, wherein the capture probe is copolymerised to the electrophoretic medium; and
(c) a third region including a collection chamber for receiving the test sample that passes through the second region, the second region separating the first region from the third region.

2. The device of Claim 1 further comprising a plurality of purification units.

3. The device of Claim 2 wherein the device is a microtiter plate and each purification unit is a microtiter well.

4. The device of any one of Claims 1, 2 and 3, wherein the collection chamber as an exit orifice.

5. The device of Claim 4 wherein the exit orifice comprises a semi-permeable membrane.

6. The device of any one of Claims 1, 2 and 3 comprising a plurality of identical capture probes.

7. The device of any one of Claims 1, 2 and 3 comprising a plurality of different capture probes.

8. A method for purifying a target nucleic acid molecule from a test sample comprising the steps of:
(a) introducing a test sample containing the target nucleic acid molecule into the receptacle of a unit of a purification device comprising:
(1) a first region including a receptacle for receiving a sample;
(2) a second region including an electrophoretic medium with at least one immobilized capture probe selected to hybridize to the target nucleic acid, wherein the capture probe is copolymerised to the electrophoretic medium; and
(3) a third region including a collection chamber for receiving of the test sample that passes through the second region, the second region separating the first region from the third region; and
(b) subjecting the electrophoretic medium to an electric field resulting in the migration of the test sample through the medium, under conditions suitable for the target molecule in the test sample to hybridize to the capture probe, thereby forming a target molecule/capture probe complex, and for the remaining components of the test sample to migrate through and elute from the medium.

9. The method of Claim 8 further comprising treating the electrophoretic medium to release the target molecule.

10. The method of Claim 9, wherein the target molecule is released from the electrophoretic medium by a treatment selected from the group consisting of raising the temperature of the electrophoretic medium to a temperature sufficient to denature the target molecule/capture probe complex, cleaving the chemical linkage which immobilizes the capture probes within the electrophoretic medium and increasing the elecuophoretic field strength to a level sufficient to disrupt the target molecule/capture probe complex.

11. The method of Claim 9 wherein the target molecule is released into the receptacle.

12. The method of any one of claims 8, 9 and 10 further comprising amplifying the target molecule.

13. The method of any one of Claims 8 to 11 wherein the sample is a stool sample.

14. The method of Claim 13, further comprising analyzing the target nucleic acid molecules to detect the presence of colorectal cancer.

15. Use for the detection of a human disease of a target nucleic acid purified by a device according to any one of Claims 1 to 7 or by a method according to any one of Claims 8 to 13, the target nucleic acid being a mutant nucleic acid.

16. Use according to Claim 15 wherein
(a) the disease is cancer; or
(b) the disease is colorectal cancer.

## Patentansprüche

1. Gerät zur Reinigung eines Target-Nukleinsäuremoleküls aus einer Untersuchungsprobe, umfassend mindestens eine Reinigungseinheit, wobei die Reinigungseinheit einen Kanal einschließt, umfassend:
(a) eine erste Zone, die ein Gefäß zum Aufnehmen einer Probe einschließt;
(b) eine zweite Zone, die ein Elektrophoresemedium mit mindestens einer immobilisierten Fangsonde, ausgewählt, um an die Target-Nukleinsäure zu hybridisieren, wobei die Fangsonde an das Elektrophoresemedium copolymerisiert ist, einschließt; und
(c) eine dritte Zone, die eine Sammelkammer zum Aufnehmen der Untersuchungsprobe, die die zweite Zone durchläuft, wobei die zweite Zone die erste Zone von der dritten Zone trennt, einschließt.

2. Gerät gemäß Anspruch 1, weiter umfassend eine Mehrzahl von Reinigungseinheiten.

3. Gerät gemäß Anspruch 2, wobei das Gerät eine Mikrotiter-Platte und jede Reinigungseinheit ein Mikrotiter-Well ist.

4. Gerät gemäß einem der Ansprüche 1, 2 und 3, wobei die Sammelkammer eine Austrittsöffnung hat.

5. Gerät gemäß Anspruch 4, wobei die Austrittsöffnung eine semipermeable Membran umfasst.

6. Gerät gemäß einem der Ansprüche 1, 2 und 3, umfassend eine Mehrzahl identischer Fangsonden.

7. Gerät gemäß einem der Ansprüche 1, 2 und 3, umfassend eine Mehrzahl unterschiedlicher Fangsonden.

8. Verfahren zur Reinigung eines Target-Nukleinsäuremoleküls aus einer Untersuchungsprobe, umfassend die Schritte:
(a) Einbringen einer Untersuchungsprobe, die das Target-Nukleinsäuremolekül enthält, in das Gefäß eines Reinigungsgerätes, umfassend
(1) eine erste Zone, die ein Gefäß zum Aufnehmen einer Probe einschließt;
(2) eine zweite Zone, die ein Elektrophoresemedium mit mindestens einer immobilisierten Fangsonde, ausgewählt, um an die Target-Nukleinsäure zu hybridisieren, wobei die Fangsonde an das Elektrophoresemedium copolymerisiert ist, einschließt; und
(3) eine dritte Zone, die eine Sammelkammer zum Aufnehmen der Untersuchungsprobe, die die zweite Zone durchläuft, wobei die zweite Zone die erste Zone von der dritten Zone trennt, einschließt; und
(b) Aussetzen des Elektrophoremediums einem elektrischen Feld, was zur Migration der Untersuchungsprobe durch das Medium führt, unter Bedingungen, die geeignet sind für das Target-Molekül in der Untersuchungsprobe, um an die Fangsonde zu hybridisieren, wodurch ein Target-Molekül/Fangsonden-Komplex gebildet wird, und für die restlichen Komponenten der Untersuchungsprobe, um hindurchzuwandern und vom Medium zu eluieren.

9. Verfahren gemäß Anspruch 8, weiter umfassend die Behandlung des Elektrophoresemediums zum Freisetzen des Target-Moleküls.

10. Verfahren gemäß Anspruch 9, wobei das Target-Molekül aus dem Elektrophoresemedium freigesetzt wird durch eine Behandlung, ausgewählt aus Erhöhung der Temperatur des Elektrophoresemediums auf eine Temperatur, die genügt, um den Target-Molekül/Fangsonden-Komplex zu denaturieren, Aufspalten der chemischen Bindung, wodurch die Fangsonden im Elektrophoresemedium immobilisiert werden, und Erhöhung der elektrophoretischen Feldstärke auf einen Wert, der genügt, um den Target-Molekül/ Fangsonden-Komplex zu spalten.

11. Verfahren gemäß Anspruch 9, wobei das Target-Molekül in das Gefäß freigesetzt wird.

12. Verfahren gemäß einem der Ansprüche 8, 9 und 10, weiter umfassend das Verstärken des Target-Moleküls.

13. Verfahren gemäß einem der Ansprüche 8 bis 11, wobei die Probe eine Stuhlprobe ist.

14. Verfahren gemäß Anspruch 13, weiter umfassend das Analysieren der Target-Nukleinsäuremoleküle, um das Vorhandensein von kolorektalem Karzinom festzustellen.

15. Verwendung einer Target-Nukleinsäure, gereinigt durch ein Gerät gemäß einem der Ansprüche 1 bis 7 oder mit einem Verfahren gemäß einem der Ansprüche 8 bis 13, wobei die Target-Nukleinsäure eine mutierte Nukleinsäure ist, zur Feststellung einer Erkrankung beim Menschen.

16. Verwendung gemäß Anspruch 15, wobei
(a) die Erkrankung Krebs ist; oder
(b) die Erkrankung kolorektales Karzinom ist.

## Revendications

1. Dispositif de purification d'une molécule d'acide nucléique cible à partir d'un échantillon de test comprenant au moins une unité de purification, l'unité de purification comprenant un conduit qui comprend :
(a) une première région comprenant un réceptacle destiné à recevoir un échantillon ;
(b) une deuxième région comprenant un support électrophorétique avec au moins une sonde de capture immobilisée choisie pour s'hybrider à l'acide nucléique cible, dans laquelle la sonde de capture est copolymérisée avec le support électrophorétique ; et
(c) une troisième région comprenant une chambre de collection destinée à recevoir l'échantillon de test qui traverse la deuxième région, la deuxième région séparant la première région de la troisième région.

2. Dispositif selon la revendication 1, comprenant en outre une pluralité d'unités de purification.

3. Dispositif selon la revendication 2, dans lequel le dispositif est une plaque de microtitration et chaque unité de purification est un puits de microtitration.

4. Dispositif selon l'une quelconque des revendications 1, 2 et 3, dans lequel la chambre de collection a un orifice de sortie.

5. Dispositif selon la revendication 4, dans lequel l'orifice de sortie comprend une membrane semi-perméable.

6. Dispositif selon l'une quelconque des revendications 1, 2 et 3, comprenant une pluralité de sondes de capture identiques.

7. Dispositif selon l'une quelconque des revendications 1, 2 et 3, comprenant une pluralité de sondes de capture différentes.

8. Procédé de purification d'une molécule d'acide nucléique cible à partir d'un échantillon de test, comprenant les étapes consistant à :
(a) introduire un échantillon de test contenant la molécule d'acide nucléique cible dans le réceptacle d'une unité d'un dispositif de purification comprenant :
(1) une première région comprenant un réceptacle destiné à recevoir un échantillon ;
(2) une deuxième région comprenant un support électrophorétique avec au moins une sonde de capture immobilisée choisie pour s'hybrider à l'acide nucléique cible, dans laquelle la sonde de capture est copolymérisée avec le support électrophorétique ; et
(3) une troisième région comprenant une chambre de collection destinée à recevoir l'échantillon de test qui traverse la deuxième région, la deuxième région séparant la première région de la troisième région, et
(b) soumettre le support électrophorétique à un champ électrique résultant en la migration de l'échantillon de test à travers le support, dans des conditions adaptées pour que la molécule cible de l'échantillon de test s'hybride à la sonde de capture, formant ainsi un complexe molécule cible/sonde de capture, et pour que les composants restants dans l'échantillon de test migrent à travers le substrat et soient élues du support.

9. Procédé selon la revendication 8, comprenant en outre le traitement du support électrophorétique pour libérer la molécule cible.

10. Procédé selon la revendication 9, dans lequel la molécule cible est libérée du support électrophorétique par un traitement choisi dans le groupe constitué d'une augmentation de la température du support électrophorétique à une température suffisante pour dénaturer le complexe molécule cible/sonde de capture, du clivage de la liaison chimique qui immobilise les sondes de capture à l'intérieur du support électrophorétique, et de l'augmentation de la force du champ électrophorétique à un niveau suffisant pour dissocier le complexe molécule cible/sonde de capture.

11. Procédé selon la revendication 9, dans lequel la molécule cible est libérée dans le réceptacle.

12. Procédé selon l'une quelconque des revendications 8, 9 et 10, comprenant en outre l'amplification de la molécule cible.

13. Procédé selon l'une quelconque des revendications 8 à 11, dans lequel l'échantillon est un échantillon de matière fécale.

14. Procédé selon la revendication 13, comprenant en outre l'analyse des molécules d'acide nucléique cibles pour détecter la présence d'un cancer colorectal.

15. Utilisation pour la détection d'une pathologie humaine d'un acide nucléique cible purifié par un dispositif selon l'une quelconque des revendications 1 à 7, ou par un procédé selon l'une quelconque des revendications 8 à 13, l'acide nucléique cible étant un acide nucléique mutant.

16. Utilisation selon la revendication 15, dans laquelle :
(a) la pathologie est le cancer ; ou
(b) la pathologie est le cancer colorectal.
